# EUROPEAN PATENT APPLICATION

(11) **EP 4 579 671 A1**
(43) Date of publication of application: **02.07.2025**
(21) Application number: 24221513.5
(22) Date of filing: 19.12.2024
(51) Int. Cl.: G16B 40/20, G16B 25/00

(54) **APPARATUS, METHOD, AND PROGRAM**

(30) Priority: 27.12.2023 JP 2023221233
(71) Applicant: Yokogawa Electric Corporation, Tokyo 180-8750 (JP)
(72) Inventor: TADENUMA, Takashi, Musashino-shi, Tokyo, 180-8750 (JP); TAGUCHI, Tomoyuki, Musashino-shi, Tokyo, 180-8750 (JP)
(74) Representative: Hoffmann Eitle

(57) **Abstract**

[Solution] Provided is an apparatus including: a detection unit which detects a position of a probe hybridized with a nucleic acid included in a specimen among a plurality of probes each of which being provided at a unique position within a biochip and at least some probes of which each having a base sequence different from one another; and a learning processing unit which performs a learning processing of a model which outputs a type of an organism having the nucleic acid included in the specimen in response to a pattern of the position of the probe hybridized with the nucleic acid included in the specimen being newly input, by using learning data including the pattern of the position of the probe hybridized with the nucleic acid included in the specimen and detected by the detection unit, and the type of the organism having the nucleic acid included in the specimen.

## Description

### BACKGROUND

### 1. TECHNICAL FIELD

The present invention relates to an apparatus, a method, and a program.

### 2. RELATED ART

For example, Patent Document 1 describes, in e.g. Paragraphs 0215 and 0316, that "1. A fluorescence probe is designed, synthesized, and purified for a target RNA. 2. Biodot is used for printing the probe (1 nM) and a surfactant, which is Zwittergent for example, on an X plate. 3. One drop of blood is applied on a substrate plate, a chip is closed and pressed, and incubated for one minute at a room temperature. 4. The chip is inserted into the device and photographed, and the data is analyzed.", "The printed probe and surfactant, which is Zwittergent for example, is dissolved in the blood. The surfactant, which is Zwittergent for example, dissolves erythrocytes and leucocytes are permeabilized to facilitate the probe entering into cells for annealing the target RNA".

### Prior Art Documents

### Patent Documents

Patent Document 1: Japanese Translation Publication of A PCT Route Patent Application No. 2021-536019
Patent Document 2: Japanese Translation Publication of A PCT Route Patent Application No. 2013-510579
Patent Document 3: Japanese Translation Publication of A PCT Route Patent Application No. 2018-508228
Patent Document 4: International Publication No. 2019/43779
Patent Document 5: Japanese Patent No. 5624487
Patent Document 6: Japanese Patent No. 6439810
Patent Document 7: Japanese Patent No. 5928906
Patent Document 8: Japanese Patent Application Publication No. 2015-42152.
Non-Patent Document 1: Tadenuma et al. "Development of a Nucleic Acid Detection System for Rapid Microbial Tests", Yokogawa Technical Report Vol. 60, 2017, Internet <URL: https://web-material3.yokogawa.com/19/13323/tabs/rd-tr-r06001-002.jp.pdf>

### SUMMARY

A first aspect of the present invention provides an apparatus, comprising: a detection unit which detects a position of a probe hybridized with a nucleic acid included in a specimen among a plurality of probes each of which being provided at a unique position within a biochip and at least some probes of which each having a base sequence different from one another; and a learning processing unit which performs a learning processing of a model which outputs a type of an organism having the nucleic acid included in the specimen in response to a pattern of the position of the probe hybridized with the nucleic acid included in the specimen being newly input, by using learning data including the pattern of the position of the probe hybridized with the nucleic acid included in the specimen and detected by the detection unit, and the type of the organism having the nucleic acid included in the specimen. All probes of the biochip may each have a base sequence different from those of other probes or some probes of the biochip may have a same base sequence as other probes. A label which emits a signal detectable by the detection unit in a hybridized state may be attached in advance to at least one of the probe or the nucleic acid included in the specimen.

In the apparatus described above, the learning processing unit may perform the learning processing of distinct models, each being the model, for each type of the biochip in which at least one of the base sequence of the probe or the unique position of the probe is different. After the learning processing of the model is performed by using some learning data, the learning processing unit may evaluate the model based on a degree of coincidence between the type of the microbe output by supplying the position pattern data of some other learning data to the model and the type of the microbe indicated by the classification data and may complete the learning processing of the model in response to the degree of coincidence being equal to or greater than a reference value.

Any of the apparatuses described above may further include: a supply unit which supplies, to the model, the pattern of the position of the probe hybridized with the nucleic acid included in one specimen and newly detected by the detection unit; and an identification unit which identifies the type of the organism output from the model in response to the pattern of the position of the probe being supplied by the supply unit as the type of the organism having the nucleic acid included in the one specimen.

A second aspect of the present invention provides an apparatus, comprising: a detection unit which detects a position of a probe hybridized with a nucleic acid included in one specimen among a plurality of probes each of which being provided at a unique position within a biochip and at least some probes of which each having a base sequence different from one another; a supply unit which supplies a pattern of the position of the probe detected by the detection unit to a model which outputs a type of an organism having the nucleic acid included in the specimen in response to the pattern of the position of the probe hybridized with the nucleic acid included in the specimen being input; and an identification unit which identifies the type of the organism output from the model in response to the pattern of the position of the probe being supplied by the supply unit as the type of the organism having the nucleic acid included in the one specimen.

In any of the apparatuses described above which includes the identification unit, the supply unit may supply the pattern of the position of the probe detected by the detection unit to the model corresponding to the biochip, the position of the probe of which being detected by the detection unit, among a plurality of models different for each type of the biochip in which at least one of the base sequence of the probe or the unique position of the probe is different, wherein each of the plurality of models is the model.

In any of the apparatuses described above, at least some of the plurality of probes may each have a base sequence common to a plurality of types of organisms.

In any of the apparatuses described above, the plurality of probes may each have a base sequence in which a number of bases is 20 or less.

A third aspect of the present invention provides a method, comprising: detecting a position of a probe hybridized with a nucleic acid included in a specimen among a plurality of probes each of which being provided at a unique position within a biochip and at least some probes of which each having a base sequence different from one another; and performing a learning processing of a model which outputs a type of an organism having the nucleic acid included in the specimen in response to a pattern of the position of the probe hybridized with the nucleic acid included in the specimen being newly input, by using learning data including the pattern of the position of the probe hybridized with the nucleic acid included in the specimen and detected by the detecting, and the type of the organism having the nucleic acid included in the specimen.

A fourth aspect of the present invention provides a method, comprising: detecting a position of a probe hybridized with a nucleic acid included in one specimen among a plurality of probes each of which being provided at a unique position within a biochip and at least some probes of which each having a base sequence different from one another; supplying a pattern of a position of a probe detected by the detecting to a model which outputs a type of an organism having the nucleic acid included in the specimen in response to the pattern of the position of the probe hybridized with the nucleic acid included in the specimen being input; and identifying a type of an organism output from the model in response to the pattern of the position of the probe being supplied by the supplying as a type of an organism having the nucleic acid included in the one specimen.

A fifth aspect of the present invention provides a program which causes a computer to function as: a detection unit which detects a position of a probe hybridized with a nucleic acid included in a specimen among a plurality of probes each of which being provided at a unique position within a biochip and at least some probes of which each having a base sequence different from one another; and a learning processing unit which performs a learning processing of a model which outputs a type of an organism having the nucleic acid included in the specimen in response to a pattern of the position of the probe hybridized with the nucleic acid included in the specimen being newly input, by using learning data including the pattern of the position of the probe hybridized with the nucleic acid included in the specimen and detected by the detection unit, and the type of the organism having the nucleic acid included in the specimen.

A sixth aspect of the present invention provides a program which causes a computer to function as: a detection unit which detects a position of a probe hybridized with a nucleic acid included in one specimen among a plurality of probes each of which being provided at a unique position within a biochip and at least some probes of which each having a base sequence different from one another; a supply unit which supplies a pattern of the position of the probe detected by the detection unit to a model which outputs a type of an organism having the nucleic acid included in the specimen in response to the pattern of the position of the probe hybridized with the nucleic acid included in the specimen being input; and an identification unit which identifies a type of an organism output from the model in response to the pattern of the position of the probe being supplied by the supply unit as a type of an organism having the nucleic acid included in the one specimen.

Note that the summary clause does not necessarily describe all necessary features of the embodiments of the present invention. The invention may also include a sub-combination of the features described above.

### BRIEF DESCRIPTION OF THE DRAWINGS

Fig. 1 illustrates an apparatus 1 according to an embodiment.
Fig. 2 illustrates a probe 101 of a biochip 100 according to an embodiment together with a transparent substrate 105 and a nucleic acid 110.
Fig. 3 illustrates operations of the apparatus 1.
Fig. 4 illustrates other operations of the apparatus 1.
Fig. 5 illustrates an example of a computer 2200 in which a plurality of aspects of the present invention may be entirely or partially embodied.

### DESCRIPTION OF EXEMPLARY EMBODIMENTS

Hereinafter, the present invention will be described through embodiments of the invention, but the following embodiments do not limit the invention according to claims. In addition, not all of the combinations of features described in the embodiments are essential to the solutions of the invention.

### <1. Apparatus>

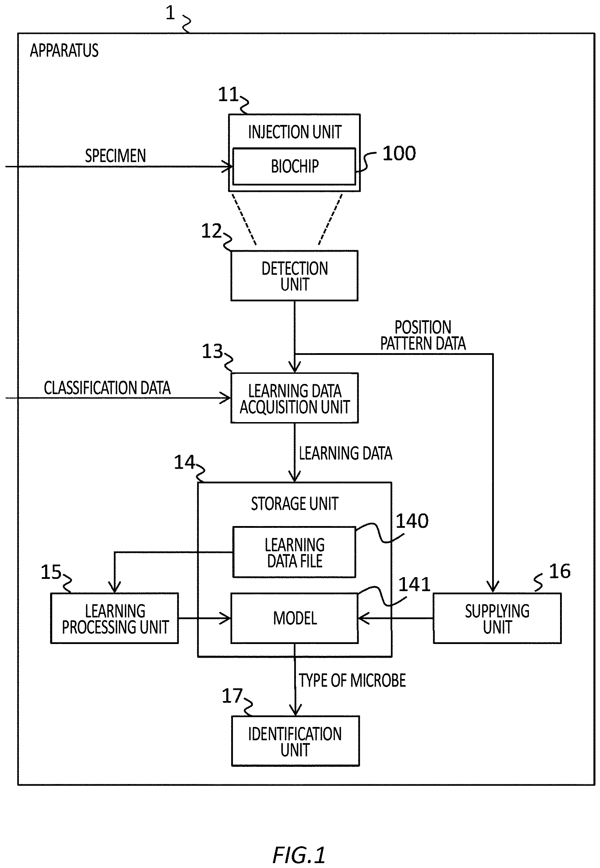

Fig. 1 illustrates an apparatus 1 according to the present embodiment. The apparatus 1 may perform a learning processing of a model 141 which identifies a type of a microbe contaminating a food or a beverage and, in addition to or instead of the above, may identify the type of the microbe contaminating the food or the beverage by using the model 141. The apparatus 1 includes an injection unit 11, a detection unit 12, a learning data acquisition unit 13, a storage unit 14, a learning processing unit 15, a supply unit 16, and an identification unit 17.

As an example, a microbe to be identified can be selected from a group consisting of Acinetobacter sp., Actinomyces sp., Aerococcus sp., Aeromonas sp., Alcaligenes sp., Bacillus sp., Bacteriodes sp., Bordetella sp., Branhamella sp., Brevibacterium sp., Campylobacter sp., Candida sp., Capnocytophaga sp., Chromobacterium sp., Clostridium sp., Corynebacterium sp., Cryptococcus sp., Deinococcus sp., Enterococcus sp., Erysipelothrix sp., Escherichia sp., Flavobacterium sp., Gemella sp., Haemophilus sp., Klebsiella sp., Lactobacillus sp., Lactococcus sp., Legionella sp., Leuconostoc sp., Listeria sp., Micrococcus sp., Mycobacterium sp., Neisseria sp., Cryptosporidium sp., Nocardia sp., Oerskovia sp., Paracoccus sp., Pediococcus sp., Peptostreptococcus sp., Propionibacterium sp., Proteus sp., Pseudomonas sp., Rahnella sp., Rhodococcus sp., Rhodospirillium sp., Staphylococcus sp., Streptomyces sp., Streptococcus sp., Vibrio sp., and Yersinia sp. While there may exist a microbe that has a form of an endospore or a spore when in an oligotrophic state, the present invention is not limited to such a state of a cell depending on a growth state.

### <1.1. Injection Unit 11>

The injection unit 11 injects a specimen into a biochip 100. A nucleic acid, which is a genome DNA, a ribosomal RNA, or a plasmid DNA as an example, of any of microbes to be identified may be included in the specimen. The specimen may be generated from a food or a beverage using a conventionally known technique. For example, the specimen may be generated by collecting a microbe included in a sample extracted from a food or a beverage via filtering or centrifugation and extracting a nucleic acid of the microbe. The collected microbe may be cultured and then subjected to nucleic acid extraction. As a technique for extracting the nucleic acid from the microbe, a technique described in International Publication No. 2019/43779 and Japanese Patent No. 5624487, which is so called a dHTP method, may be used and, as an example, the nucleic acid may be extracted by housing the microbe captured on a filter in a container together with the filter and breaking a membrane structure of the microbe by exposing the microbe to a high temperature condition. The extracted nucleic acid may be amplified by a technique of PCR (polymerase chain reaction).

The injection unit 11 according to the present embodiment may inject the specimen into a same type of biochips 100. Each of the biochips 100 has a plurality of probes inside. At least some probes of the plurality of probes may each have a base sequence different from one another. As an example, all probes of the biochip 100 may each have a base sequence different from those of other probes or some probes of the biochip 100 may have a same base sequence as other probes.

At least some of the plurality of probes included in the biochip 100 may have a base sequence common to a plurality of types of microbes. When the biochip 100 has a probe having a base sequence that is not common to the plurality of types of microbes, the base sequence may be a base sequence unique to a microbe to be identified or may be a base sequence not included in the microbe to be identified. However, in a designing stage of the biochip 100, a base sequence of each probe may be decided independently of a base sequence of a nucleic acid of the microbes to be identified. As an example, the base sequence of each probe may be decided at random provided that the base sequences of each probe are the same among the same type of biochips 100. Each probe may include a base sequence in which a number of bases is 20 or less. As an example, a number of the probes each having the base sequence different from one another may be 100 or more.

Each probe may be hybridized with a corresponding base sequence. At least some probes of the plurality of probes may each have a base sequence complementary to at least some base sequences of the nucleic acid of the microbes to be identified and may be specifically hybridized with the nucleic acid having a corresponding base sequence.

The biochip 100 may further have a positive control probe which emits a signal to be detected by a detection unit 12 independently of the nucleic acid included in the specimen. The positive control probe may not be hybridized with the nucleic acid.

Each probe and the positive control probe may be provided at a unique position within the same type of biochips 100. For example, a probe at a same position may have a same base sequence in the same type of biochips 100. In the present embodiment, as an example, the biochip 100 may have a plurality of probes and one or more positive control probes on inner surfaces of two transparent substrates arranged to oppose one another. The biochip 100 may have an inlet for injecting the specimen inside and an outlet for discharging a liquid contained inside.

A label which emits a signal detectable by the detection unit 12, which will be described below, in a hybridized state may be attached in advance to at least one of the probe of the biochip 100 or the nucleic acid included in the specimen. For example, the label may be attached to the probe and may emit the signal in response to hybridization of the probe and the nucleic acid. Instead of the above, the label may be attached to the nucleic acid of the specimen and may emit the signal regardless of whether the nucleic acid is hybridized with the probe. In this case, after the specimen is injected into the biochip 100, detection of the signal may be performed after the nucleic acid that is not hybridized with the probe is removed from the biochip 100. For example, a fluorescent dye, a radioisotope or a paramagnetic isotope, or an enzyme can be used as the label. In the present embodiment, as an example, the label may be the fluorescent dye and may be attached to the probe. The label may also be attached in advance to the positive control probe. The label of the positive control probe may emit the signal at all times.

Because the at least some probes of the biochip 100 according to the present embodiment each have the base sequence common to the plurality of types of microbes, a microbe, which is also referred to as a contaminating microbe, having the nucleic acid included in the specimen may not be identified by only checking whether individual probes are hybridized. Meanwhile, because the at least some probes of the biochip 100 each have the base sequence different from one another, a position pattern of hybridized probes may be different according to a type of the contaminating microbe. Therefore, by using the biochip 100 according to the present embodiment, the type of the contaminating microbe according to the position pattern of the hybridized probes may be identified by learning the position pattern of the hybridized probes and the type of the contaminating microbe.

### <1.2. Detection Unit 12>

The detection unit 12 detects a position of the probe hybridized with the nucleic acid included in the specimen among the plurality of probes of the biochip 100. The detection unit 12 may detect the position of the hybridized probe with reference to a position of the positive control probe. The detection unit 12 may detect the hybridized probe or the positive control probe by detecting the label within the biochip 100. The detection unit 12 may detect the signal emitted from the label attached to any one of the hybridized probe or the nucleic acid. The detection unit 12 may further detect the signal emitted from the positive control probe. The detection unit 12 may detect the label within the biochip 100 while its position is fixed relative to the biochip 100. The detection unit 12 may be arranged to face the biochip 100 and may detect the label within the biochip 100 via a transparent substrate of the biochip 100. The detection unit 12 may supply, to the learning data acquisition unit 13 and the supply unit 16, data indicating a pattern of a position of a detected label, which is also referred to as position pattern data. The position pattern data may include information indicating a strength of the signal at each detected position. When the detection unit 12 detects fluorescence from the label as the signal, the position pattern data may include information indicating a wavelength of the signal at each detected position.

### <1.3. Learning Data Acquisition Unit 13>

The learning data acquisition unit 13 acquires learning data including the pattern of the position of the probe hybridized with the nucleic acid included in the specimen and detected by the detection unit 12, and the type of the microbe having the nucleic acid included in the specimen. The learning data may be data used for learning of the model 141. The microbe having the nucleic acid included in the specimen may be a microbe from which the nucleic acid included in the specimen is extracted and may be a microbe included in the food or the beverage from which the specimen is generated.

The learning data acquisition unit 13 may generate the learning data by associating the position pattern data indicating the position pattern of the probe hybridized with the nucleic acid included in the specimen and detected with data indicating the type of the microbe from which the nucleic acid is extracted, which is also referred to as classification data. The learning data acquisition unit 13 may acquire the position pattern data from the detection unit 12 and may acquire the classification data from an operator. As an example, at a learning stage of the model 141, a specimen including a nucleic acid of any microbe may be generated and injected into the biochip 100 by an operator, and the classification data of the microbe may be supplied from the operator to the learning data acquisition unit 13 and the position pattern data of the probe hybridized with the nucleic acid of the microbe may be supplied from the detection unit 12 to the learning data acquisition unit 13. The learning data acquisition unit 13 may acquire, for example, from the operator, the learning data generated in advance by associating the position pattern data with the classification data. The learning data acquisition unit 13 may supply the acquired learning data to the storage unit 14.

### <1.4. Storage Unit 14>

The storage unit 14 stores a variety of information. The storage unit 14 may store a learning data file 140 and the model 141.

### <1.4.1. Learning Data File 140>

The learning data file 140 stores the learning data. In the present embodiment, as an example, the learning data file 140 may store each learning data supplied from the learning data acquisition unit 13.

### <1.4.2. Model 141>

The model 141 outputs the type of the contaminating microbe of the specimen in response to the pattern of the position of the probe hybridized with the nucleic acid included in the specimen being newly input. In the present embodiment, as an example, the model 141 may output the type of the microbe in response to the position pattern data output from the detection unit 12 being input. The model 141 may undergo the learning processing by the learning processing unit 15. As an example, the model 141 may be an image analysis engine.

### <1.5. Learning Processing Unit 15>

The learning processing unit 15 performs the learning processing of the model 141. The learning processing unit 15 performs the learning processing on the model 141 in the storage unit 14 by reading the learning data from the learning data file 140 of the storage unit 14. The learning processing unit 15 may perform the learning processing of the model 141 by machine learning such as deep learning.

### <1.6. Supply Unit 16>

The supply unit 16 supplies, to the model 141, the pattern of the position of the probe hybridized with the nucleic acid included in one specimen and newly detected by the detection unit 12. The supply unit 16 may supply, to the model 141 which underwent the learning processing by the learning processing unit 15, the position pattern data supplied from the detection unit 12. Thus, data indicating the type of the contaminating microbe may be output from the model 141 to the identification unit 17.

### <1.7. Identification Unit 17>

The identification unit 17 identifies the type of the microbe output from the model 141 in response to the position pattern data being supplied to the model 141 by the supply unit 16 as the type of the contaminating microbe of the one specimen described above. The identification unit 17 may output information indicating the type of the contaminating microbe to an outside.

According to the apparatus 1 described above, the learning processing of the model 141 is performed by using the learning data including the position pattern of the probe detected in response to hybridization with the nucleic acid included in the specimen and the type of the microbe having the nucleic acid included in the specimen. Thus, the model 141 which outputs the type of the contaminating microbe in response to the position pattern of the hybridized probe being input can be generated. As a result, cost of the biochip 100 can be reduced because the type of the contaminating microbe can be identified in response to the position pattern of the hybridized probe being input to the model 141 without preparing a probe having a unique base sequence for each microbe.

The type of the microbe output from the model 141 is identified as the type of the contaminating microbe in response to the pattern of the position of the probe newly detected by the detection unit 12 being supplied to the model 141. Thus, the type of the contaminating microbe can be identified by the position of the hybridized probe being detected by the detection unit 12.

Because the at least some of the plurality of probes of the biochip 100 each have the base sequence common to the plurality of types of microbes, the base sequence of the probe can be shortened compared to a case where the plurality of probes have a unique base sequence for each microbe. Thus, cost of the biochip 100 can be reduced.

Because the plurality of probes of the biochip 100 each have the base sequence in which the number of bases is 20 or less, cost of the biochip 100 can be reduced compared to a case where each have a base sequence where the number of bases is more than 20.

### <2. Probe of Biochip 100>

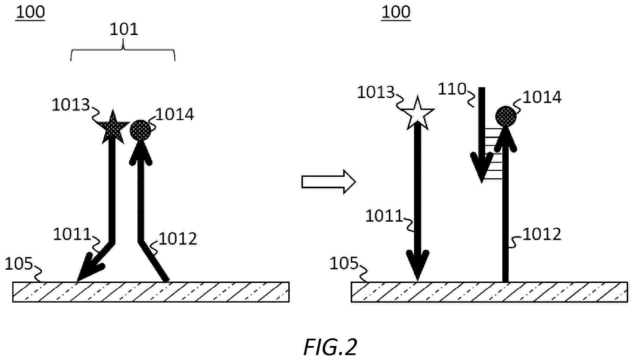

Fig. 2 illustrates the probe 101 of the biochip 100 according to the present embodiment together with the transparent substrate 105 and the nucleic acid 110. A black arrow in the figure indicates the probe or the nucleic acid. A head of the arrow indicates a 5' end and a shaft of the arrow indicates a 3' end.

Each probe 101 may have a first probe 1011 and a second probe 1012 each having a base sequence complementary to one another. The first probe 1011 may be a fluorescence probe modified with a fluorescent dye 1013 and the second probe 1012 may be a quenching probe modified with a quencher 1014 which reduces light emission of the fluorescent dye. As illustrated on a left-hand side in the figure, when a target nucleic acid does not exist within the biochip 100, the light emission of the fluorescent dye 1013 of the first probe 1011 may be reduced because the first probe 1011 and the second probe 1012 are hybridized with one another so that the fluorescent dye 1013 of the first probe 1011 and the quencher of the second probe 1012 approach one another. As illustrated on a right-hand side in the figure, when a target nucleic acid 110 exists within the biochip 100, the fluorescent dye 1013 of the first probe 1011 may emit light because at least one of the first probe 1011 or the second probe 1012 is hybridized with the target nucleic acid 110 so that the fluorescent dye 1013 of the first probe 1011 and the quencher 1014 of the second probe 1012 are separated from one another. In the biochip 100 having such a probe, after the specimen is injected into the biochip 100, detection of the label can be performed without removing, for example, the nucleic acid that is not hybridized with the probe 101 from the biochip 100. For example, those described in Japanese Patent No. 6439810, Japanese Patent No. 5928906, Japanese Patent Application Publication No. 2015-42152, or Document 1 as follows can be used as the biochip 100 described above.

Document 1: Tadenuma et al. "Development of a Nucleic Acid Detection System for Rapid Microbial Tests", Yokogawa Technical Report Vol. 60, 2017, Internet <URL: https://web-material3.yokogawa.com/19/13323/tabs/rd-tr-r06001-002.jp.pdf>

### <3. Operations of Apparatus 1>

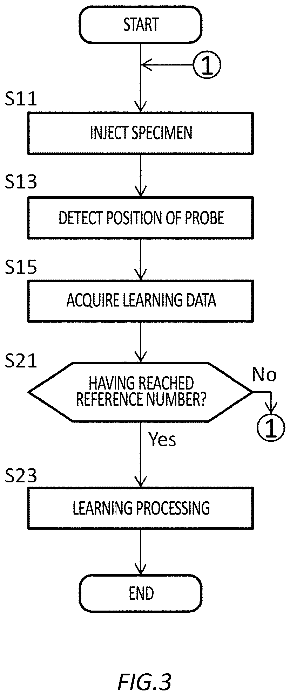

Fig. 3 illustrates operations of the apparatus 1. The apparatus 1 performs the learning processing of the model 141 by performing processes of step S11 to S23.

In step S11, the injection unit 11 injects the specimen into the biochip 100. The injected specimen may include a nucleic acid of one or more microbes arbitrarily selected by the operator among the microbes to be identified.

In step S13, the detection unit 12 detects the position of the probe hybridized with the nucleic acid included in the specimen among the plurality of probes of the biochip 100. Thus, the position of the probe hybridized with the nucleic acid of the microbe included in the specimen among the probes each having the base sequence different one another may be detected.

In step S15, the learning data acquisition unit 13 acquires learning data including the pattern of the position of a probe hybridized with the nucleic acid included in the specimen and detected by the detection unit 12 and a type of the microbe having the nucleic acid included in the specimen. The learning data acquisition unit 13 may acquire the position pattern data from the detection unit 12 and acquire the classification data of the microbe from an operator. The classification data may indicate the type of a single microbe or may indicate the type of the plurality of microbes.

In step S21, the learning data acquisition unit 13 determines whether a number of learning data has reached a reference number. The reference number may be set arbitrarily. When the number of learning data has not reached the reference number (step S21; No), the process may proceed to step S11. When the number of learning data has reached the reference number (step S23; Yes), the process may proceed to step S23.

In step S23, the learning processing unit 15 performs the learning processing of the model 141 by using the learning data acquired. Thus, the model 141 which outputs the type of one or more contaminating microbes in response to the pattern of the position of the hybridized probe being input is generated.

The learning processing unit 15 may perform a performance evaluation of the model 141 by performing a cross-validation. For example, after the learning processing of the model 141 is performed by using some learning data in the storage unit 14, the learning processing unit 15 may evaluate the model 141 based on a degree of coincidence between the type of the microbe output by supplying the position pattern data of some other learning data to the model 141 and the type of the microbe indicated by the classification data. The learning processing unit 15 may complete the learning processing of the model 141 in response to the degree of coincidence being equal to or greater than a reference value. The learning processing unit 15 may cause the process to proceed to step S11 or may output an error signal and then end the process, in response to the degree of coincidence being lower than the reference value. The error signal may prompt the operator to redo, using another type of biochip, the operations of step S11 to S23 on a new model 141.

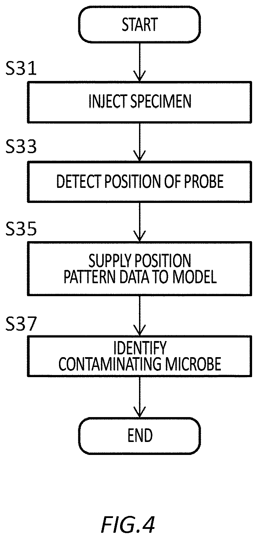

Fig. 4 illustrates other operations of the apparatus 1. The apparatus 1 identifies the contaminating microbe using the model 141 which underwent the learning processing by performing the processes of step S31 to S37.

In step S31, the injection unit 11 injects one specimen into the biochip 100. The injected specimen may include the nucleic acid of the one or more microbes among the microbes to be identified.

In step S33, similarly to step S13, the detection unit 12 detects the position of the probe hybridized with the nucleic acid included in the one specimen among the plurality of probes of the biochip 100.

In step S35, the supply unit 16 supplies, to the model 141, the pattern of the position of the probe detected by the detection unit 12 in step S33. Thus, data indicating the type of the one or more contaminating microbes may be output from the model 141.

In step S37, the identification unit 17 identifies the type of the microbe output from the model 141 as the type of the contaminating microbes of the one specimen. The identification unit 17 may output information indicating the type of the contaminating microbe to an outside. When the process of step S37 ends, the apparatus 1 may complete the operation or may cause the process to proceed to step S31 described above.

### <4. Variant>

While the apparatus 1 includes the injection unit 11, the learning data acquisition unit 13, the storage unit 14, the learning processing unit 15, the supply unit 16 and the identification unit 17 in the description of the embodiment above, the apparatus 1 may not include any of the above. For example, when the apparatus 1 does not include the supply unit 16 and the identification unit 17, the learning of the model 141 may be performed by the learning processing unit 15 and a learned model 141 may be output. When the apparatus 1 does not include the learning processing unit 15, the apparatus 1 may perform identification of a contaminating microbe by using the learned model 141. When the apparatus 1 does not include the storage unit 14, the apparatus 1 may perform the learning processing on the model 141 within an externally connected storage device or may perform the identification of the contaminating microbe by using the model 141 in the externally connected storage device.

While the injection unit 11 injects the specimen into the same type of biochips 100 in the description above, the specimen may be injected into a plurality of types of biochips 100 in which at least one of a base sequence of the probe or a unique position of the probe is different. In this case, the storage unit 14 may store the model 141 and learning data file 140 which are different for each type of the biochip 100, and the learning data acquisition unit 13 may acquire, from the operator, the types of the biochips 100 used and store the learning data in the learning data file 140 corresponding to the types of the biochips 100 used.

When the specimen is injected into the plurality of types of biochips 100, the learning processing unit 15 may perform the learning processing on distinct models 141 for each type of the biochip 100. As a result, lowering of learning accuracy can be prevented, unlike a case where the learning processing of a same model 141 is performed by using different types of biochips 100. The organism in the specimen can be identified by using a variety of biochips 100.

When the specimen is injected into the plurality of types of biochips 100, the supply unit 16 may supply the position pattern data to the model 141 corresponding to the biochip 100 of which the position of the probe is detected by the detection unit 12, that is, the biochip 100 used, among a plurality of different models 141 for each type of the biochip 100. Thus, the microbe in the specimen can be identified by using a variety of biochips 100.

While the model 141 which outputs the type of the microbe having the nucleic acid included in the specimen is described above, a type of one or more organisms other than the microbe may be output. In this case, the organism to be identified may be an animal, an insect, a plant, a mycoplasma or a virus.

In addition, various embodiments of the present invention may be described with reference to flowcharts and block diagrams, wherein the block may serve as (1) a stage in a process in which an operation is performed, or (2) a section of an apparatus having a role of performing an operation. Certain stages and sections may be implemented by a dedicated circuit, a programmable circuit supplied together with computer-readable instructions stored on computer-readable media, and/or processors supplied together with computer-readable instructions stored on computer-readable media. The dedicated circuit may include digital and/or analog hardware circuits, and may include integrated circuits (IC) and/or discrete circuits. The programmable circuit may include a reconfigurable hardware circuit including logical AND, logical OR, logical XOR, logical NAND, logical NOR, and other logical operations, a memory element or the like such as a flip-flop, a register, a field programmable gate array (FPGA) and a programmable logic array (PLA), or the like.

A computer-readable medium may include any tangible device that can store instructions to be executed by a suitable device, and as a result, the computer-readable medium having instructions stored thereon includes a product including instructions that can be executed in order to create means for executing operations specified in the flowcharts or block diagrams. Examples of the computer-readable medium may include an electronic storage medium, a magnetic storage medium, an optical storage medium, an electromagnetic storage medium, a semiconductor storage medium, and the like. More specific examples of the computer-readable medium may include a floppy (registered trademark) disk, a diskette, a hard disk, a random access memory (RAM), a read-only memory (ROM), an erasable programmable read-only memory (EPROM or flash memory), an electrically erasable programmable read-only memory (EEPROM), a static random access memory (SRAM), a compact disc read-only memory (CD-ROM), a digital versatile disk (DVD), a Blu-ray (registered trademark) disk, a memory stick, an integrated circuit card, or the like.

The computer-readable instruction may include: an assembler instruction, an instruction-set-architecture (ISA) instruction; a machine instruction; a machine dependent instruction; a microcode; a firmware instruction; state-setting data; or either a source code or an object code described in any combination of one or more programming languages, including an object oriented programming language such as Smalltalk (registered trademark), JAVA (registered trademark), C++, or the like, and a conventional procedural programming language such as a "C" programming language or a similar programming language.

Computer-readable instructions may be provided to a processor of a general-purpose computer, special purpose computer, or other programmable data processing apparatuses, or to a programmable circuit, locally or via a local area network (LAN), wide area network (WAN) such as the Internet, or the like, so that the computer-readable instructions are executed to create means for performing operations specified in the flowcharts or block diagrams. Examples of the processor include a computer processor, a processing unit, a microprocessor, a digital signal processor, a controller, a microcontroller, and the like.

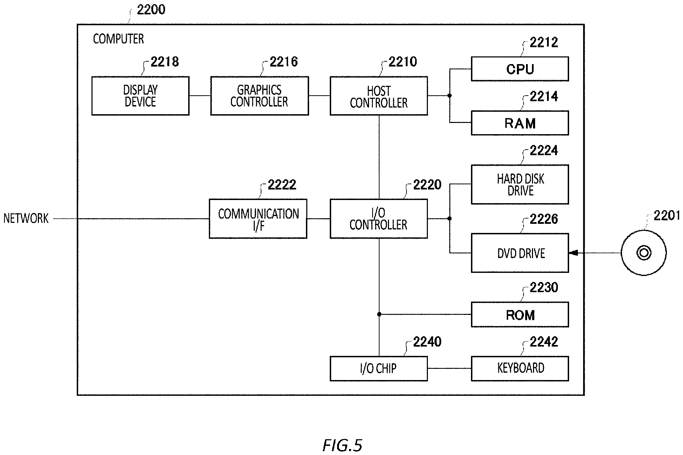

Fig. 5 illustrates an example of a computer 2200 in which a plurality of aspects of the present invention may be entirely or partially embodied. A program installed in the computer 2200 can cause the computer 2200 to function as an operation associated with the apparatuses according to the embodiments of the present invention or as one or more sections of the apparatuses, or can cause the operation or the one or more sections to be executed, and/or can cause the computer 2200 to execute a process according to the embodiments of the present invention or a stage of the process. Such programs may be executed by a CPU 2212 to cause the computer 2200 to perform specific operations associated with some or all of the blocks in the flowcharts and block diagrams described in the present specification.

The computer 2200 according to the present embodiment includes the CPU 2212, an RAM 2214, a graphics controller 2216, and a display device 2218, which are mutually connected by a host controller 2210. The computer 2200 also includes input/output units such as a communication interface 2222, a hard disk drive 2224, a DVD-ROM drive 2226, and an IC card drive, which are connected to the host controller 2210 via an input/output controller 2220. The computer also includes legacy input/output units such as an ROM 2230 and a keyboard 2242, which are connected to the input/output controller 2220 via an input/output chip 2240.

The CPU 2212 operates according to programs stored in the ROM 2230 and the RAM 2214, thereby controlling each unit. The graphics controller 2216 acquires image data generated by the CPU 2212 in a frame buffer or the like provided in the RAM 2214 or in itself, such that the image data is displayed on the display device 2218.

The communication interface 2222 communicates with other electronic devices via a network. The hard disk drive 2224 stores programs and data used by the CPU 2212 in the computer 2200. The DVD-ROM drive 2226 reads a program or data from a DVD-ROM 2201 and provides the program or data to the hard disk drive 2224 via the RAM 2214. The IC card drive reads the programs and the data from the IC card, and/or writes the programs and the data to the IC card.

The ROM 2230 stores therein boot programs and the like executed by the computer 2200 at the time of activation, and/or programs that depend on the hardware of the computer 2200. The input/output chip 2240 may also connect various input/output units to the input/output controller 2220 via a parallel port, a serial port, a keyboard port, a mouse port, or the like.

Programs are provided by a computer-readable medium such as the DVD-ROM 2201 or the IC card. The programs are read from the computer-readable medium, are installed in the hard disk drive 2224, the RAM 2214, or the ROM 2230 which is also an example of the computer-readable medium, and are executed by the CPU 2212. The information processing described in these programs is read by the computer 2200, and provides cooperation between the programs and the various types of hardware resources. The apparatus or method may be configured by implementing operations or processings of information according to use of the computer 2200.

For example, in a case where communication is performed between the computer 2200 and an external device, the CPU 2212 may execute a communication program loaded in the RAM 2214 and instruct the communication interface 2222 to perform communication processing based on a processing written in the communication program. Under the control of the CPU 2212, the communication interface 2222 reads transmission data stored in a transmission buffer processing area provided in a recording medium such as the RAM 2214, the hard disk drive 2224, the DVD-ROM 2201, or the IC card, transmits the read transmission data to the network, or writes reception data received from the network in a reception buffer processing area or the like provided on the recording medium.

In addition, the CPU 2212 may cause the RAM 2214 to read all or a necessary part of a file or database stored in an external recording medium such as the hard disk drive 2224, the DVD-ROM drive 2226 (DVD-ROM 2201), the IC card, or the like, and may execute various types of processing on data on the RAM 2214. Then, the CPU 2212 writes the processed data back in the external recording medium.

Various types of information such as various types of programs, data, tables, and databases may be stored in a recording medium and subjected to information processing. The CPU 2212 may execute, on the data read from the RAM 2214, various types of processing including various types of operations, information processing, conditional judgement, conditional branching, unconditional branching, information retrieval/replacement, or the like described throughout the present disclosure and specified by instruction sequences of the programs, and writes the results back to the RAM 2214. In addition, the CPU 2212 may retrieve information in a file, a database, or the like in the recording medium. For example, when a plurality of entries, each having an attribute value of a first attribute associated with an attribute value of a second attribute, is stored in the recording medium, the CPU 2212 may retrieve, out of the plurality of entries, an entry with the attribute value of the first attribute specified that meets a condition, read the attribute value of the second attribute stored in said entry, and thereby acquiring the attribute value of the second attribute associated with the first attribute meeting a predetermined condition.

The programs or software modules described above may be stored in a computer-readable medium on or near the computer 2200. In addition, a recording medium such as a hard disk or an RAM provided in a server system connected to a dedicated communication network or the Internet can be used as a computer-readable medium, thereby providing a program to the computer 2200 via the network.

While the present invention has been described with the embodiments, the technical scope of the present invention is not limited to the above-described embodiments. It is apparent to persons skilled in the art that various modifications or improvements can be added to the above-described embodiments. It is also apparent from description of the claims that the embodiments to which such modifications or improvements are made can be included in the technical scope of the present invention.

It should be noted that the operations, procedures, steps, stages, and the like of each process performed by an apparatus, system, program, and method shown in the claims, the specification, or the drawings can be realized in any order as long as the order is not indicated by "prior to," "before," or the like and as long as the output from a previous process is not used in a later process. Even if the operation flow is described using phrases such as "first" or "next" for the sake of convenience in the claims, specification, and drawings, it does not mean that the process must be performed in this order.

### EXPLANATION OF REFERENCES

1: apparatus
11: injection unit
12: detection unit
13: learning data acquisition unit
14: storage unit
15: learning processing unit
16: supply unit
17: identification unit
100: biochip
101: probe
105: transparent substrate
110: nucleic acid
140: learning data file
141: model
1011: first probe
1012: second probe
1013: fluorescent dye
1014: quencher
2200: computer
2201: DVD-ROM
2210: host controller
2212: CPU
2214: RAM
2216: graphics controller
2218: display device
2220: input/output controller
2222: communication interface
2224: hard disk drive
2226: DVD-ROM drive
2230: ROM
2240: input/output chip
2242: keyboard.

## Claims

1. An apparatus, comprising:
a detection unit which detects a position of a probe hybridized with a nucleic acid included in a specimen among a plurality of probes each of which being provided at a unique position within a biochip and at least some probes of which each having a base sequence different from one another; and
a learning processing unit which performs a learning processing of a model which outputs a type of an organism having the nucleic acid included in the specimen in response to a pattern of the position of the probe hybridized with the nucleic acid included in the specimen being newly input, by using learning data including the pattern of the position of the probe hybridized with the nucleic acid included in the specimen and detected by the detection unit, and the type of the organism having the nucleic acid included in the specimen.

2. The apparatus according to claim 1, wherein the learning processing unit performs the learning processing of distinct models, each being the model, for each type of the biochip in which at least one of the base sequence of the probe or the unique position of the probe is different.

3. The apparatus according to claim 1 or 2, further comprising:
a supply unit which supplies, to the model, the pattern of the position of the probe hybridized with the nucleic acid included in one specimen and newly detected by the detection unit; and
an identification unit which identifies the type of the organism output from the model in response to the pattern of the position of the probe being supplied by the supply unit as the type of the organism having the nucleic acid included in the one specimen.

4. An apparatus, comprising:
a detection unit which detects a position of a probe hybridized with a nucleic acid included in one specimen among a plurality of probes each of which being provided at a unique position within a biochip and at least some probes of which each having a base sequence different from one another;
a supply unit which supplies a pattern of the position of the probe detected by the detection unit to a model which outputs a type of an organism having the nucleic acid included in the specimen in response to the pattern of the position of the probe hybridized with the nucleic acid included in the specimen being input; and
an identification unit which identifies the type of the organism output from the model in response to the pattern of the position of the probe being supplied by the supply unit as the type of the organism having the nucleic acid included in the one specimen.

5. The apparatus according to claim 3 or 4, wherein the supply unit supplies the pattern of the position of the probe detected by the detection unit to the model corresponding to the biochip, the position of the probe of which being detected by the detection unit, among a plurality of models different for each type of the biochip in which at least one of the base sequence of the probe or the unique position of the probe is different, wherein each of the plurality of models is the model.

6. The apparatus according to any one of claims 1 to 5, wherein at least some of the plurality of probes each have a base sequence common to a plurality of types of organisms.

7. The apparatus according to any one of claims 1 to 6, wherein the plurality of probes each have a base sequence in which a number of bases is 20 or less.

8. A method, comprising:
detecting a position of a probe hybridized with a nucleic acid included in a specimen among a plurality of probes each of which being provided at a unique position within a biochip and at least some probes of which each having a base sequence different from one another; and
performing a learning processing of a model which outputs a type of an organism having the nucleic acid included in the specimen in response to a pattern of the position of the probe hybridized with the nucleic acid included in the specimen being newly input, by using learning data including the pattern of the position of the probe hybridized with the nucleic acid included in the specimen and detected by the detecting, and the type of the organism having the nucleic acid included in the specimen.

9. A method, comprising:
detecting a position of a probe hybridized with a nucleic acid included in one specimen among a plurality of probes each of which being provided at a unique position within a biochip and at least some probes of which each having a base sequence different from one another;
supplying a pattern of a position of a probe detected by the detecting to a model which outputs a type of an organism having the nucleic acid included in the specimen in response to the pattern of the position of the probe hybridized with the nucleic acid included in the specimen being input; and
identifying a type of an organism output from the model in response to the pattern of the position of the probe being supplied by the supplying as a type of an organism having the nucleic acid included in the one specimen.

10. A program which causes a computer to function as:
a detection unit which detects a position of a probe hybridized with a nucleic acid included in a specimen among a plurality of probes each of which being provided at a unique position within a biochip and at least some probes of which each having a base sequence different from one another; and
a learning processing unit which performs a learning processing of a model which outputs a type of an organism having the nucleic acid included in the specimen in response to a pattern of the position of the probe hybridized with the nucleic acid included in the specimen being newly input, by using learning data including the pattern of the position of the probe hybridized with the nucleic acid included in the specimen and detected by the detection unit, and the type of the organism having the nucleic acid included in the specimen.

11. A program which causes a computer to function as:
a detection unit which detects a position of a probe hybridized with a nucleic acid included in one specimen among a plurality of probes each of which being provided at a unique position within a biochip and at least some probes of which each having a base sequence different from one another;
a supply unit which supplies a pattern of the position of the probe detected by the detection unit to a model which outputs a type of an organism having the nucleic acid included in the specimen in response to the pattern of the position of the probe hybridized with the nucleic acid included in the specimen being input; and
an identification unit which identifies a type of an organism output from the model in response to the pattern of the position of the probe being supplied by the supply unit as a type of an organism having the nucleic acid included in the one specimen.
